# EUROPEAN PATENT APPLICATION

(11) **EP 3 272 348 A1**
(43) Date of publication of application: **24.01.2018**
(21) Application number: 16180606.2
(22) Date of filing: 21.07.2016
(51) Int. Cl.: A61K 31/517, A61K 47/10, A61K 47/12, A61K 47/18, A61K 47/36, A61K 47/38, A61K 9/20, A61K 31/52, A61P 19/08, A61P 35/02

(54) **PHARMACEUTICAL COMPOSITION COMPRISING IDELALISIB**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to a new pharmaceutical composition comprising idelalisib, or a salt or a prodrug or a solvate thereof, and pharmaceutically acceptable excipients, said excipients providing efficient idelalisib release from final dosage form in gastric and post-gastric conditions. The present invention relates to a new process for the preparation of pharmaceutical composition comprising idelalisib or a salt or a prodrug or a solvate thereof, or a salt, or a prodrug, or a solvate thereof. The pharmaceutical composition is particularly useful as a medicament, especially for the treatment of cancers of hematopoietic origin.

## Description

### Field of the Invention

The present invention relates to a pharmaceutical composition of idelalisib, or a salt or prodrug or a solvate thereof, and to a process for the preparation of pharmaceutical composition comprising amorphous idelalisib, or a salt or prodrug or a solvate thereof. The pharmaceutical composition is particularly useful as a medicament for inhibiting phosphatydyl inositol 3-kinase delta, as a medicament for disrupting osteoclast function, as a medicament for ameliorating a bone resorption disorder and as a medicament for the treatment of a cancer of hemapoietic origin.

### Description of Background Art

Idelalisib ((S)-2-(I-(9H-purin-6-ylamino)propyl)-5-fluoro-3-phenyl-quinazolin-4(3H)-one having structural formula (I) was disclosed in WO 2001/81346 and WO 2005/113556.

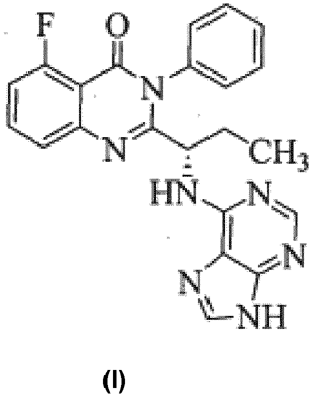

Idelalisib is a compound that selectively modulates human phosphoinositide 3 -kinase (PI3K) delta activity, and thereby promotes medical treatment of diseases mediated by PI3K delta dysfunction. More specifically idelalisib is an investigational, targeted, highly selective oral inhibitor of phosphoinositide 3 -kinase (PI3K) delta, a protein that is critical for the inflammatory functions of neutrophils, for the endogenous immune response stimulated by at least one endogenous factor, for leukocyte accumulation to the site of insult or inflamed tissue, for lymphocyte proliferation and for antibody production by B lymphocyte.

Idelalisib is being developed as a medicament for treating a medical condition mediated by neutrophils; for treating a medical condition wherein a function of osteoclasts is disrupted; for ameliorating a bone-resorption disorder in a mammal in need thereof; for inhibiting the growth or proliferation of cancer cells of hematopoietic origin, more specifically as a medicament for inhibiting the growth or proliferation of cancers selected from the group consisting of lymphomas, multiple myelomas, and leukaemia. Details concerning the methods of using idelalisib for selectively modulating PI3K delta activity in clinical setting are disclosed in WO 2005/1135556 and are incorporated herein as a reference. Zydeig^{®} (idelalisib) was approved by US FDA for Relapsed chronic lymphocytic leukaemia (CLL), Relapsed follicular B-cell non-Hodgkin lymphoma (FL) and Relapsed small lymphocytic lymphoma (SLL).

Various crystalline polymorphic forms of idelalisib, Form I, Form II, Form III, Form IV, Form V, Form VI and Form VIII, and pharmaceutical composition comprising one or more polymorphic forms of ((S)-2-(I-(9H-purin-6-ylamino)propyl)-5-fluoro-3-phenyl-quinazolin-4(3H)-one and a pharmaceutically acceptable carrier has been disclosed in WO 2013/134288.

Crystalline idelalisib demonstrates pH dependent solubility; solubility decreases with increasing pH. Crystalline idelalisib is not soluble at pH 7 and soluble at pH 1.2 and shows high permeability. Therefore it is classified by biopharmaceutical classification system as class 2 (BCS class II). Most of BCS class II drugs generally exhibit higher bioavailability if their release from the pharmaceutical final dosage form (FDF) is increased.

Preparations of amorphous idelalisib substance as such, and of a pharmaceutical composition comprising an amorphous solid dispersion of idelalisib in a suitable polymer, obtained from a solution of idelalisib and the polymer in an organic solvent and subsequently removing the solvent, are disclosed in WO 2015/092810.

In general amorphous forms of active pharmaceutical ingredients exhibit higher aqueous solubility in comparison to corresponding crystalline forms, thereby possibly having higher bioavailability. Which form among various crystalline and amorphous forms is preferably presented in a pharmaceutical composition or FDF, or which technical treatment or formulation process of a specifically given active pharmaceutical ingredient will be suitable in terms of solubility and bioavailability - i.e. dealing not only with solubility of the active pharmaceutical ingredient (API) as such, but also and in particular with release behaviour from a FDF containing the API - however cannot be predicted. This would particularly apply if a pH dependent solubility behaviour of the active ingredient compound was involved.

We have found that, although conventional inactive pharmaceutical ingredients (excipients) including binders, disintegrants and lubricants had been used, pharmaceutical composition (designed as FDF) comprising idelalisib may have mutually opposite pH-dependent solubility behavior depending on the idelalisib form: while amorphous idelalisib showed high idelalisib release from FDF in media with neutral pH, but low idelalisib release from FDF in acidic media, pharmaceutical compositions comprising crystalline idelalisib showed low idelalisib release from FDF in media with neutral pH and high idelalisib release from FDF in acidic media. In case of idelalisib, such pH-related inhibition of idelalisib release from a pharmaceutical composition designed as an FDF comprising idelalisib is highly undesirable.

The data from open-label trial with 100 mg and 150 mg tablets comprising crystalline idelalisib (brand name Zydeig^{®}) performed by Gilead demonstrated decreased bioavailability and clinical efficiency of the drug at pH of 6 or higher. With respect to WO 2015/092810 in which only amorphous idelalisib and amorphous solid dispersion comprising idelalisib are disclosed, due to the nature of solid dispersions and the polymers used for the solid dispersion preparations, slower dissolution, especially in acidic media, is to be expected.

Therefore, there was an unmet need for a new pharmaceutical composition comprising idelalisib, or a salt or a prodrug or a solvate thereof, that exhibit high idelalisib release from FDF in both, gastric and post-gastric conditions.

### Summary of the invention

The present invention satisfies this need. In the present invention, it has been surprisingly found that when selecting amorphous idelalisib, or a salt or a prodrug or a solvate thereof, and presenting it in a pharmaceutical composition in which its amorphous status is surely and stably achieved and maintained, it is possible to obtain a highly advantageous idelalisib release behaviour, which includes a significantly improved release from FDF in acidic media compared to the standard amorphous reference FDF product, without detriment to a same or similarly high release from FDF in neutral media where crystalline idelalisib would be critically affected in dissolution. Accordingly, the present invention provides a pharmaceutical composition of idelalisib in the form of an FDF which beneficially utilizes a constantly high dissolution under both gastric conditions occurring in the stomach as well as post-gastric conditions occurring in the intestine, thereby providing an overall excellent bioavailability behavior.

More specifically, the present invention provides a pharmaceutical composition comprising amorphous idelalisib, or a salt or a prodrug or a solvate thereof, with comparable idelalisib release from FDF in acidic media to pharmaceutical compositions comprising crystalline idelalisib, or a salt or a prodrug or a solvate thereof, without however encountering the disadvantage of too low idelalisib release from FDF in neutral media. Even more specifically, the present invention provides a pharmaceutical composition in the form of an FDF comprising amorphous idelalisib, or a salt or a prodrug or a solvate thereof, with at least 60% of the total amount of idelalisib release from FDF in neutral media, and at the same time with at least 60% of the total amount of idelalisib release from FDF in acidic media, thereby exhibiting high idelalisib release from FDF in acidic media and in media with neutral pH and thus exhibiting improved bioavailability in terms of overall considering both gastric and post-gastric conditions.

The aspects, advantageous features and preferred embodiments of the present invention summarized in the following items, respectively alone or in combination, further contribute to solving the object of the invention:
1. Pharmaceutical composition of idelalisib in the form of an FDF comprising idelalisib, or a salt or a prodrug or a solvate thereof, and one or more pharmaceutically acceptable excipient, wherein said idelalisib, or a salt or a prodrug or a solvate thereof, is in amorphous form, and wherein the pharmaceutical composition provides for an idelalisib release property defined by at least 60% of the total amount of idelalisib release from FDF when measured in each of acidic media and media with neutral pH.
2. The pharmaceutical composition according to item 1, wherein said idelalisib release property defined by at least 60% of the total amount of idelalisib release from FDF in each of acidic media and media with neutral pH is respectively met after 10 minutes in 0.1 HCl and after 60 minutes at pH of about 6.4.
3. The pharmaceutical composition according to item 1 or 2, wherein said idelalisib release property to be met in each of said acidic media and neutral media is defined by at least 70%, preferably at least 80% of the total amount of idelalisib release from FDF.
4. The pharmaceutical composition according to anyone of the preceding claims, wherein said idelalisib release property of the pharmaceutical composition is provided for by selecting the pharmaceutically acceptable excipients from the groups consisting of cellulose derivatives, aromatic amino acids, and polyols.
5. The pharmaceutical composition according to item 4, wherein the cellulose derivative excipient is selected from hydroxypropylcellulose, hydroxypropylmethylcellulose, methylcellulose, ethylcellulose, hydroxyethylcellulose and carboxymethylcellulose, preferably selected from hydroxypropylcellulose and hydroxypropylmethylcellulose; the aromatic amino acid excipient is phenylalanine and the polyol excipient is mannitol.
6. The pharmaceutical composition according to anyone of the preceding items, wherein said idelalisib release property of the pharmaceutical composition is provided for by containing a mechanically intimately mixed association of amorphous idelalisib, or a salt or a prodrug or a solvate thereof, with the one or more pharmaceutically acceptable excipient.
7. The pharmaceutical composition according to item 6, wherein the mechanically intimately mixed association is formed by high-shear mixing, preferably by co-milling (ball milling, impact milling) of amorphous idelalisib, or a salt, or a prodrug, or a solvate thereof with the one or more pharmaceutically acceptable excipient, preferably wherein the co-milling is formed with an excipient selected from the groups consisting of cellulose derivatives and polyols, more preferably the cellulose derivative is selected from hydroxypropyl cellulose and hydroxypropylmethyl cellulose, and the polyol excipient is mannitol.
8. The pharmaceutical composition according to anyone of the preceding items, wherein said idelalisib release property of the pharmaceutical composition is provided for by containing a granulate obtained by wet granulating of amorphous idelalisib, or a salt or a prodrug or a solvate thereof, or a mixture of amorphous idelalisib, or a salt or a prodrug or a solvate thereof, and at least one pharmaceutically acceptable excipient, with a liquid or a solution of pharmaceutically acceptable excipient(s).
9. The pharmaceutical composition according to anyone of the preceding items, comprising further (optionally other, different) pharmaceutically acceptable excipients selected from disintegrants, binders, fillers, and lubricants.
10. The pharmaceutical composition according to item 9, wherein said disintegrant is selected from the group consisting agar-agar, algines, alginic acid, alginates, calcium carbonate, sodium alginate, carboxymethylcellulose, calcium carboxymethylcellulose, sodium carboxymethylcellulose, cellulose, clays, colloidal silicone dioxide, croscarmellose sodium, crospovidone, gums, magnesium aluminium silicate, cellulose derivates, methylcellulose, microcrystalline cellulose, maltose, low substituted hydroxypropyl cellulose, crosslinked polyvinylpyrrolidone, polacrilin potassium, starch, starch derivatives, pregelatinized starch, pregelatinized starch and povidone.
11. The pharmaceutical composition according to item 9, wherein said binder is selected from the group consisting of agar; cellulose derivatives, preferably carboxy methyl cellulose, calcium/sodium carboxymethylcellulose, hydroxypropylmethyl cellulose, hydroxypropyl cellulose, hydroxymethyl cellulose, hydroxyethyl cellulose, ethyl cellulose; corn syrup, dextrose, gelatin, gelatin hydrolysate, glucose, lactose, maltose, methylcellulose, microcrystalline cellulose, PEG, polymeric cellulose derivatives, polyethylene glycol polymethacrylates, polyvinylpyrrolidone, povidone, pregelatinized starch, sodium alginate, sorbitol, starch paste, starch, sucrose, sugar syrups, sugar alcohols, vinyl pyrrolidone copolymers, water soluble polysaccharides such as acacia, tragacanth, guar and alginates.
12. The pharmaceutical composition according to item 9, wherein said filler is selected from the group consisting of calcium carbonate, calcium phosphate, dibasic calcium phosphate, tribasic calcium sulfate, calcium hydrogen phosphate, calcium sulfate, calcium carboxymethylcellulose, cellulose, citric acid, dextrates, lactose monohydrate, dextrin, dextrose, fructose, lacticol, directly compressible starch, hydrolyzed starches, lactose, magnesium carbonate, magnesium oxide, mannitol, maltitol, maltodextrins, maltose, microcrystalline cellulose, potassium chloride, sodium chloride, sorbitol, starch, spray-dried lactose, sucrose, sugar, sucrose-based materials, xylitol.
13. The pharmaceutical composition according to item 9, wherein said lubricant is selected from the group consisting of agar, calcium stearate, ethyl oleate, ethyl laureate, glycerine, glyceryl palmitostearate, glyceryl behenate, hydrogenated vegetable oils (e.g. hydrogenated castor oil), magnesium oxide, magnesium stearate, mannitol, poloxamer, glycols, polyethylene glycols, sodium benzoate, sodium lauryl sulfate, sodium stearyl, sodium stearyl fumarate, stearic acid salts, stearic acid derivatives, sorbitol, stearic acid, zinc stearate, talc, and waxes.
14. The pharmaceutical composition according to any of preceding items, comprising relative to the total amount of the formulation:
   (i) about 10 - 85% amorphous idelalisib, or a salt or a prodrug or a solvate thereof,
   (ii) about 1 - 30% excipients selected from cellulose derivatives, aromatic amino acids and polyols, wherein preferably the cellulose derivative is selected from hydroxypropyl cellulose and hydrohypropylmethyl cellulose; the aromatic amino acid preferably is phenylalanine; and polyol preferably is mannitol.
   (iii) about 0 - 30% disintegrant, preferably croscarmellose
   (iv) about 0 - 30% binder, preferably low-substituted hydroxypropyl cellulose and sodium starch glycolate,
   (v) about 0 - 30% filler, preferably microcrystalline cellulose,
   (vi) about 0.2 - 3% lubricant, preferably magnesium stearate.
15. The pharmaceutical composition according to any one of the preceding items, obtained by direct compression.
16. A granulate obtained by wet granulating of amorphous idelalisib, or a salt or a prodrug or a solvate thereof, or a mixture of amorphous idelalisib, or a salt or a prodrug or a solvate thereof, and at least one pharmaceutically acceptable excipient, with a liquid or a solution of pharmaceutically acceptable excipient(s).
17. The granulate according to item 16, wherein the pharmaceutically acceptable excipient is preferably cellulose derivate selected from hydroxypropylcellulose, hydroxypropylmethylcellulose, methylcellulose, ethylcellulose, hydroxyethylcellulose and carboxymethylcellulose, preferably is selected from hydroxypropyl cellulose and hydroxypropylmethyl cellulose.
18. A mixture of amorphous idelalisib, or a salt or a prodrug or a solvate thereof, co-milled with one or more pharmaceutically acceptable excipient selected from the groups consisting of cellulose derivatives and polyols.
19. The mixture according to item 18, wherein the cellulose derivative is selected from hydroxypropylcellulose, hydroxypropylmethylcellulose, methylcellulose, ethylcellulose, hydroxyethylcellulose and carboxymethylcellulose, preferably is selected from hydroxypropyl cellulose and hydroxypropylmethyl cellulose; and wherein the polyol is mannitol.
20. Use of the granulate according to item 16 or 17, or of a mixture according to item 18 or 19, for preparing a final dosage form (FDF) of a pharmaceutical composition comprising idelalisib, or a salt or a prodrug or a solvate thereof.
21. A process for preparing a pharmaceutical composition according to anyone of the preceding items, comprising
   (i) mixing the amorphous idelalisib, or a salt or a prodrug or a solvate thereof, with at least one said pharmaceutically acceptable excipients under a condition of absence of any liquid;
   (ii) compressing the mixture.
22. A process for preparing a pharmaceutical composition according to anyone of the preceding items, comprising:
   (i) mixing the amorphous idelalisib, or a salt or a prodrug or a solvate thereof, with at least one pharmaceutically acceptable excipient under a condition of absence of any liquid;
   (ii) compressing the mixture by direct compression; or
      (i') mechanically intimately mixing, preferably grinding, the amorphous idelalisib, or a salt or a prodrug or a solvate thereof, with the one or more pharmaceutically acceptable excipient;
      (ii') mixing the mixture obtained in (i') with further pharmaceutically acceptable excipient, optionally selected from disintegrants, binders, fillers, and lubricants under a condition of absence of any liquid;
      (iii') compressing the mixture; or

      (i") granulating the amorphous idelalisib, or a salt or a prodrug or a solvate thereof, or a mixture obtained under (i) with a liquid or a solution of pharmaceutically acceptable excipient(s) to form wet granulate;
      (ii") mixing the mixture obtained in (i") with at least one further pharmaceutically acceptable excipient selected from disintegrants, binders, fillers, and lubricants;
      (iii") compressing the mixture.
23. The process according to item 22, wherein the excipient of (i) is selected from cellulose derivatives, aromatic amino acids, and polyols, and wherein the excipient of (i') or (i") is selected from cellulose derivatives; preferably the cellulose derivative excipient is selected from hydroxypropylcellulose, hydroxypropylmethylcellulose, methylcellulose, ethylcellulose, hydroxyethylcellulose and carboxymethylcellulose, more preferably is selected from hydroxypropyl cellulose and hydroxypropylmethyl cellulose, the aromatic amino acid is phenylalanine, and the polyol is mannitol.
24. The process according to item 22, wherein the mechanically intimately mixing of (i') is high-shear mixing, preferably is co-milling.
25. The process according to anyone of items 22 to 24, wherein the mixture is compressed and tableted into tablet cores.
26. The process according to item 25 wherein the tablet cores are further coated with coating containing polymer selected from the group consisting of polyvinyl alcohol, hydroxypropyl methylcellulose, and hydroxypropyl cellulose.
27. The pharmaceutical composition according to anyone of items 1 to 15 for use in a method for treatment of disrupted osteoclast function, of bone resorption disorder and of cancers of hematopoietic origin.

### Description of further advantages and preferred embodiments of the invention

The present invention is now described in more detail by preferred embodiments and examples, which are however presented for illustrative purpose only.

The object of the present invention was to provide pharmaceutical composition comprising idelalisib, or a salt or a prodrug or a solvate thereof, with improved release of idelalisib from FDF in gastric (low pH) and post-gastric conditions (neutral pH) and with improved bioavailability.

If not otherwise specified, the term "idelalisib" means the compound of formula I specified above specifically, or it means generally the compound itself, or a salt or a prodrug or a solvate thereof.

One aspect the present invention provides a pharmaceutical composition of idelalisib which is a final dosage form (FDF) comprising idelalisib, or a salt or a prodrug or a solvate thereof, wherein said idelalisib, or a salt or a prodrug or a solvate thereof, is in amorphous form, and wherein the pharmaceutical composition has one or more pharmaceutically acceptable excipient(s) which provide(s) for an idelalisib release property defined by at least 60% of the total amount of idelalisib release from FDF when measured in each of acidic media and media with neutral pH.

The release may be carried out under standard testing methods, for example by using standard paddle apparatus (Apparatus 2) with agitation of 50 RPM. The volume of the media can be 900mL. Acidic media can be represented by 0,1M HCL, while neutral media can be represented by FaSSIF buffer at pH of about 6.4.

The term "FDF" used herein means final dosage form, more specifically it means immediate release dosage form. Suitable FDFs are tablets, capsules, caplets and lozenges, preferably tablets. The FDF obtained in the present invention is capable of releasing idelalisib under both, gastric and post-gastric conditions. The immediate release dosage form may include a coating which imparts additional properties, such as taste-masking coating that dissolves to release drug in the stomach.

The term "release of idelalisib from FDF in acidic media" used herein means release of the idelalisib from the FDF under conditions corresponding to, or equal to, gastric conditions in stomach. For ease of measuring, such release in acidic media is suitably determined under controlled laboratory environment, for example by using standard paddle apparatus (Apparatus 2) with agitation of 50 RPM; more specifically it suitably means the percent of released idelalisib from FDF after 10 minutes in acidic media, for example in 900ml solution of 0.1M HCl, which can properly simulate gastric conditions where a testing time point of 10 minutes well corresponds to quick transfer of pharmaceutical compositions through the stomach.

The term "release of idelalisib from FDF in media with neutral pH" used herein means release of the idelalisib from the FDF under conditions corresponding to, or equal to, post-gastric conditions, typically present in the intestine after the stomach. For ease of measuring, such release in media with neutral pH is suitably determined under controlled laboratory environment, for example by using standard paddle apparatus (Apparatus 2) with agitation of 50 RPM, more specifically it suitably means the percent of released idelalisib from FDF after 60 minutes in a buffer with pH of about 6.4, for example in 900ml FaSSIF buffer, which can properly simulate post-gastric conditions where a testing time point of 60 minutes well characterizes post-gastric conditions due to relatively long residence time in the small intestine.

It was surprisingly found that release of idelalisib from a pharmaceutical composition in the form of an FDF comprising idelalisib, or a salt or a prodrug or a solvate thereof, demonstrated an overall improvement for both acidic media and media with neutral pH, when idelalisib, or a salt or a prodrug or a solvate thereof, is chosen to be in amorphous form and when pharmaceutically acceptable excipients are chosen in view of, alone or in combination, (i) type of excipient, (ii) how amorphous idelalisib, or a salt or a prodrug or a solvate thereof, is associated with an excipient, and (iii) how the pharmaceutical composition or an intermediate thereof, respectively containing amorphous idelalisib, or a salt or a prodrug or a solvate thereof, with pharmaceutically acceptable excipient, is prepared.

As to (i), i.e. a selection of pharmaceutically acceptable excipient capable of providing for the idelalisib release property, it was found that selection is most effective if chosen from the groups consisting of cellulose derivatives, aromatic amino acids, and polyols.

Preferably, the cellulose derivatives are selected from hydroxypropylcellulose, hydroxypropylmethylcellulose, methylcellulose, ethylcellulose, hydroxyethylcellulose and carboxymethylcellulose, more preferably is selected from hydroxypropyl cellulose and hydrohypropylmethyl cellulose; the aromatic amino acid preferably is phenylalanine; and the polyol excipient preferably is mannitol. Selected pharmaceutically acceptable excipients even allow the preparation of a FDF in the form of a tablet by simple direct compression, yet meeting the desired high release properties of the FDF under both acidic and neutral conditions.

As to (ii) and (iii), it was additionally found that choosing an appropriate pre-treatment of amorphous idelalisib, or a salt or a prodrug or a solvate thereof, prior to preparing the FDF with further excipients, with an pharmaceutically acceptable excipient surprisingly can have a further significant influence on whether a release of idelalisib from the FDF at sufficiently high level at both acidic and neutral conditions for simulating gastric and post-gastric conditions is met or not. In this case, an appropriate pre-treatment determines and positively influences how amorphous idelalisib, or a salt or a prodrug or a solvate thereof, is associated with an pharmaceutically acceptable excipient.

According to one embodiment, improvement was found when amorphous idelalisib, or a salt or a prodrug or a solvate thereof, was mechanically intimately/intensely mixed with at least one pharmaceutically acceptable excipient, in particular if co-milled together with e.g. ball mill or impact mill (hammer mill). Another suitable means to mechanically intimately/intensely mix is high-shear mixing. Preferably, approaches (i) and (ii) are combined and the pharmaceutically acceptable excipient(s) to be used for the mechanically intimately/intensely mixed association with amorphous idelalisib, or a salt or a prodrug or a solvate thereof, is selected from cellulose derivatives, aromatic amino acids, and polyols and particularly from the preferred examples mentioned above.

According to a further embodiment, improvement was found when amorphous idelalisib, or a salt or a prodrug or a solvate thereof, was associated with at least one pharmaceutically acceptable excipient in a granulate obtained by wet granulating of the amorphous idelalisib, or a salt or a prodrug or a solvate thereof, or a mixture of amorphous idelalisib, or a salt or a prodrug or a solvate thereof, and at least one pharmaceutically acceptable excipient with a liquid or a solution of pharmaceutically acceptable excipient(s), preferably with a polymer solution wherein polymer is selected from hydroxypropylcellulose, hydroxypropylmethylcellulose, methylcellulose, ethylcellulose, hydroxyethylcellulose and/or carboxymethylcellulose, more preferably is hydroxypropyl cellulose and/or hydrohypropylmethyl cellulose. Distinct from a process for preparing a solid dispersion or solid solution, according to this embodiment of the invention the amorphous idelalisib does not, at least not completely dissolve in the polymer solution, and the amorphous idelalisib form can be maintained throughout its processing. Accordingly, in the present embodiment the solution is mainly, essentially or even only aqueous-based; when an organic solvent is used or used partly besides water for providing the polymer solution, it is chosen in type and amount such that the amorphous idelalisib is not dissolved in the polymer solution.

Further in comparison with a solid dispersion or solid solution, the concept of the present invention allows to use, in case a polymer such as cellulose derivative is used, a relative lower amount of polymer in relation to idelalisib, which for instance can be defined by a weight ratio of idelalisib to polymer of higher than 1 : 1, preferably using a ratio of at least 2 : 1 and higher. Dissolution and especially uniform dissolution under both gastric and post-gastric conditions can thereby be further enhanced.

In either case, the mechanically intimately co-mixed association or the granulate can subsequently be used to prepare the desired FDF, for example and preferably by compression into a tablet.

Prior direct compression and tableting other conventional pharmaceutically acceptable excipients, selected from disintegrants, binders, fillers, and lubricants, can be added to the mechanically intimately co-mixed association or to a powder mixture.

Resulting tablet cores can be further coated with coating containing polymer selected from the group consisting of polyvinyl alcohol, hydroxypropyl methylcellulose, hydroxypropyl cellulose to obtain FDF.

In a particularly preferred embodiment, wet granulation uses the amorphous idelalisib, or a salt or a prodrug or a solvate thereof, and pharmaceutically acceptable excipients selected from cellulose derivatives, preferably hydroxypropylcellulose, hydroxypropylmethylcellulose, methylcellulose, ethylcellulose, hydroxyethylcellulose and/or carboxymethylcellulose, more preferably is hydroxypropyl cellulose and/or hydrohypropylmethyl cellulose, which involves wetting amorphous idelalisib, or a salt or a prodrug or a solvate thereof, or a mixture of amorphous idelalisib, or a salt or a prodrug or a solvate thereof, and pharmaceutically acceptable excipients, such as cellulose derivatives, with a liquid, such as water or an aqueous solution, which optionally may contain a water-miscible organic solvent to an extend not to dissolve or completely dissolve the amorphous idelalisib, and then performing granulation. The wet granulation is preferably carried out with aqueous solution as liquid medium. Wet granulate is dried by conventional processes known in the art.

A step of granulation may be processed by compacting large mass of any of the described mixtures being present in larger sized pieces and subsequently crushing and sizing these pieces into smaller granules.

A step of high-shear mixing may use a high rotation shear apparatus. A suitable high-shear mixing can be carried out at a paddle speed of at least 100, for example at a paddle speed of 100 to 1500, preferably of 300 to 1200 RPM, and more preferably of 500 to 1000 RPM.

A step of grinding suitably involves milling between two surfaces. Milling can be conducted with a mortar and pestle or using a milling apparatus and process, such as ball milling, roller milling, screen milling, impact milling or gravatory milling.

In another aspect, the present invention further provides the afore-described co-milled mixture and granulate obtained by wet granulating as useful intermediates for preparing a suitable range of final dosage forms. Accordingly, the present invention provides a pharmaceutical composition that is obtainable from the afore-defined wet granulation or co-milled/ground formulation by direct compression or direct tableting, which may form final tablets or tablet cores subsequently coated, or may form granules which can be filled in capsules or in sachets.

The term "direct compression" used herein means a preparation wherein the amorphous idelalisib, or a salt or a prodrug or a solvate thereof, and further ingredients for a pharmaceutical dosage form have been processed by applying of a sufficient force by the punches of a tablet press on a powder to compact it into a tablet.

When preparing FDFs according to the present invention the above-defined pharmaceutical compositions according to the previous aspects may use further pharmaceutically acceptable excipients, suitably selected from conventional and general purpose functional excipients, such as pharmaceutically acceptable excipients selected from disintegrants, binders, fillers, and lubricants.

The term "pharmaceutically acceptable excipients" as used herein includes any physiologically inert additives that are routinely used in pharmaceutical dosage forms. Pharmaceutically acceptable excipients are selected from the group comprising binders, disintegrants, lubricants, glidants, coating additives or combinations thereof.

Accordingly, the pharmaceutical composition according to the previous aspects can further contain a filler. The term "filler" as used herein is defined as an agent used in order to achieve the desired pharmaceutical form volume or weight. The filler may be present in the pharmaceutical composition in the form of a single compound or in the form of a mixture of compounds. Various suitable fillers include but are not limited to calcium carbonate, calcium phosphate, dibasic calcium phosphate, tribasic calcium sulfate, calcium hydrogen phosphate, calcium sulfate, calcium carboxymethylcellulose, cellulose, citric acid, dextrates, lactose monohydrate, dextrin, dextrose, fructose, lacticol, directly compressible starch, hydrolyzed starches, lactose, magnesium carbonate, magnesium oxide, mannitol, maltitol, maltodextrins, maltose, microcrystalline cellulose, potassium chloride, sodium chloride, sorbitol, starch, spray-dried lactose, sucrose, sugar, sucrose-based materials, xylitol. Preferably, a microcrystalline cellulose is used as the filler.

The pharmaceutical composition described herein according to the previous aspects may also comprise a binder. The term "binder" as used herein is defined as an agent able to bind particles which cannot be bound only by a compression force. The binder may be present in the pharmaceutical composition in the form of a single compound or in the form of a mixture of compounds. Various suitable binders include but are not limited to agar, cellulose derivatives (e.g. carboxy methyl cellulose, calcium/sodium carboxymethylcellulose hydroxypropylmethyl cellulose, hydroxypropyl cellulose, hydroxymethyl cellulose, hydroxyethyl cellulose, ethyl cellulose ), corn syrup, dextrose, gelatin, gelatin hydrolysate, glucose, lactose, maltose, methylcellulose, microcrystalline cellulose, PEG, polymeric cellulose derivatives, polyethylene glycol polymethacrylates, polyvinylpyrrolidone, povidone, sodium starch glycolate, sodium alginate, sorbitol, starch paste, starch, sucrose, sugar syrups, sugar alcohols, vinyl pyrrolidone copolymers, water soluble polysaccharides such as acacia, tragacanth, guar and alginates. Preferably low-substituted hydroxypropyl cellulose and sodium starch glycolate are used as the binders.

The pharmaceutical composition described herein according to the previous aspects may also comprise disintegrants. The term "disintegrant" refers to any material that has wicking and/or swelling properties when it comes in contact with water and is used as an accelerating agent of the disintegration of the tablet and the dispersion of the active ingredient in water or gastrointestinal fluids. The disintegrator may be present in the pharmaceutical composition in the form of a single compound or in the form of a mixture of compounds. Various suitable disintegrants include but are not limited to agar-agar, algines, alginic acid, alginates, calcium carbonate, sodium alginate, carboxymethylcellulose, calcium carboxymethylcellulose, sodium carboxymethylcellulose, cellulose, clays, colloidal silicone dioxide, croscarmellose (croscarmellose sodium), crospovidone, gums, magnesium aluminium silicate, cellulose derivates, methylcellulose, microcrystalline cellulose, maltose, low substituted hydroxypropyl cellulose, crosslinked polyvinylpyrrolidone, polacrilin potassium, starch, starch derivatives, sodium starch glycolate, sodium starch glycolate and povidone. Preferably, croscarmellose is used as the disintegrant.

The pharmaceutical composition described herein according to the previous aspects may also comprise lubricants. The term "lubricant" as used herein is defined as an agent able to decrease adhesion of a powder to punches and friction between particles. The lubricant may be present in the pharmaceutical composition in the form of a single compound or in the form of a mixture of compounds. Various suitable lubricants include but are not limited to agar, calcium stearate, ethyl oleate, ethyl laureate, glycerine, glyceryl palmitostearate, glyceryl behenate, hydrogenated vegetable oils (e.g. hydrogenated castor oil), magnesium oxide, magnesium stearate, mannitol, poloxamer, glycols, polyethylene glycols, sodium benzoate, sodium lauryl sulfate, sodium stearyl, sodium stearyl fumarate, stearic acid salts, stearic acid derivatives, sorbitol, stearic acid, zinc stearate, talc, and waxes. Preferably, magnesium stearate is used as the lubricant.

The present invention further provides a set of particular embodiments of pharmaceutical compositions according to the previous aspects, respectively comprising:
about 10 - 85%, preferably 70-80% amorphous idelalisib, or a salt or a prodrug or a solvate thereof,
about 1 - 30%, preferably 5 - 15% excipients selected from cellulose derivatives, aromatic amino acids and polyol,
about 0 - 30%, preferably 4 - 7% disintegrant,
about 0 - 30%, preferably 5 - 20% binder,
about 0 - 30%, preferably 2 - 15% filler,
about 0.2 - 3%, preferably 0.5 - 1.5% lubricant.

The % indications mean weight-%.

The term "about" generally means within 10%, preferably 5% and more preferably within 1% of a given value or range. Alternatively, the term "about" means within an acceptable standard error of the mean, when considered by one of the ordinary skill in the art.

In further, particularly preferred embodiment the pharmaceutical composition comprises
about 10 - 85% amorphous idelalisib,
about 1 - 30% of excipients selected from hydroxypropylcellulose,
hydroxypropylmethylcellulose, methylcellulose, ethylcellulose, hydroxyethylcellulose, carboxymethylcellulose (more preferably selected from hydroxypropyl cellulose and hydrohypropylmethyl cellulose), phenylalanine and mannitol,
about 0 - 30% croscarmellose,
about 0 - 30% low-substituted hydroxypropyl cellulose and/or sodium starch glycolate, about 0 - 30% microcrystalline cellulose,
about 0.2 - 3% magnesium stearate.

In these embodiments of the pharmaceutical composition, a specifically suitable amount of amorphous idelalisib, or a salt or a prodrug or a solvate thereof, is about 75%, corresponding e.g. an amount of 75mg in case of an FDF total weight of 100mg, or an amount of 150mg in case of an FDF total weight of 200mg.

The pharmaceutical composition according to the previous aspects may further comprises a coating using a polymer selected from the group consisting of polyvinyl alcohol, hydroxypropyl methylcellulose, hydroxypropyl cellulose, hydroxyethylcellulose and polymethacrylates.

The term "coating" as used herein refers to a layer which completely covers an object and is applied by film coating. The coating can be selected from the group of ready to form dispersion such as OPADRY. The coating dispersion may comprise hydrophilic film forming polymer (such as for example low viscosity HPMC, HPC, PVA (polyvinylalcohol) and the like), plastificators (e.g. PEG), colorants and may optionally include other excipients such as antitacking agents. In the preferred embodiment the coating is OPADRY.

Any method for film coating, known in the field of the pharmaceutical technology, may be used.

A pharmaceutical composition according to the present invention is preferably in solid form, including tablets, capsules, caplets, lozenges and sachets. Tablets may be suitably coated (film coated tablets, pills). Capsule formulations may cover both soft and hard capsules. A pharmaceutical composition according to the present invention is preferably in the form of tablet, more preferably coated tablet with appropriate film coating material.

In another aspect, the present invention further provides a process for preparing the pharmaceutical composition comprising idelalisib or a salt or prodrug or a solvate thereof.

In a preferred embodiment of the process pharmaceutically acceptable excipients selected from hydroxypropylcellulose, hydroxypropylmethylcellulose, methylcellulose, ethylcellulose, hydroxyethylcellulose, carboxymethylcellulose (more preferably hydroxypropyl cellulose and/or hydrohypropylmethyl cellulose), phenylalanin and mannitol are first combined with amorphous idelalisib, or a salt or a prodrug or a solvate thereof, by a method selected from mixing under a condition of absence of any liquid, wet granulation or co-grinding before direct compression.

Surprisingly, we have observed that grinding amorphous idelalisib, or a salt or a prodrug or a solvate thereof, with pharmaceutically acceptable excipients, particularly if the excipients are selected from cellulose derivatives (preferably hydroxypropylcellulose, hydroxypropylmethylcellulose, methylcellulose, ethylcellulose, hydroxyethylcellulose and carboxymethylcellulose, in particular hydroxypropyl cellulose and/or hydrohypropylmethyl cellulose) and polyols (in particular mannitol), to form a homogenous mixture before direct compression with further pharmaceutically acceptable excipient selected from disintegrants, binders, fillers, and lubricants, resulted in pharmaceutical dosage form which, compared to conventional comparison products, exhibited improved release performance and higher idelalisib release from FDF in both acidic media and media with neutral pH.

Further we surprisingly observed that granulating amorphous idelalisib, or a salt or a prodrug or a solvate thereof, or a mixture of amorphous idelalisib, or a salt or a prodrug or a solvate thereof, and at least one pharmaceutically acceptable excipient with a liquid or a solution of pharmaceutically acceptable excipient(s) to form wet granulate, wherein the pharmaceutically acceptable excipient is selected from the groups consisting of cellulose derivatives, more preferably the cellulose derivative is selected from hydroxypropylcellulose, hydroxypropylmethylcellulose, methylcellulose, ethylcellulose, hydroxyethylcellulose and carboxymethylcellulose, in particular is selected from hydroxypropylcelulose and hydrohypropylmethylcelulose before direct compression with further pharmaceutically acceptable excipient selected from disintegrants, binders, fillers, and lubricants resulted in pharmaceutical dosage form exhibiting high idelalisib release from FDF in acidic media and in media with neutral pH.

A further aspect of the process according to the present invention comprises mixing the amorphous idelalisib, or a salt or a prodrug or a solvate thereof, with pharmaceutically acceptable excipients, preferably selected from hydroxypropyl cellulose, hydrohypropylmethyl cellulose, phenylalanin or mannitol under a condition of absence of any liquid and compressing the mixture with further pharmaceutically acceptable excipient selected from disintegrants, binders, fillers, and lubricants.

A particularly preferred process according to the present invention comprises wet granulating amorphous idelalisib, or a salt or a prodrug or a solvate thereof, or a mixture of amorphous idelalisib, or a salt or a prodrug or a solvate thereof, and at least one pharmaceutically acceptable excipient, with a liquid or a solution of pharmaceutically acceptable excipient(s), wherein the pharmaceutically acceptable excipient is preferably cellulose derivate selected from hydroxypropylcellulose, hydroxypropylmethylcellulose, methylcellulose, ethylcellulose, hydroxyethylcellulose and carboxymethylcellulose, preferably is selected from hydroxypropyl cellulose and hydroxypropylmethyl cellulose; mixing the obtain granulate with pharmaceutically acceptable excipient selected from disintegrants, binders, fillers, and lubricants; and compressing the mixture.

Even more preferably the process according to the present invention comprises grinding amorphous idelalisib, or a salt or a prodrug or a solvate thereof, with pharmaceutically acceptable excipients selected from hydroxypropyl cellulose, hydrohypropylmethyl cellulose, phenylalanin or mannitol to form a homogenous mixture; mixing the obtained grinding mixture with pharmaceutically acceptable excipient selected from disintegrants, binders, fillers, and lubricants under a condition of absence of any liquid; and compressing the mixture.

In another aspect, the present invention further provides a process for preparing the pharmaceutical composition further comprising coating of the compressed mixture with a coating mixture containing polymer selected from the group consisting of polyvinyl alcohol, hydroxypropyl methylcellulose, hydroxypropyl cellulose.

In another aspect, the present invention further provides the pharmaceutical composition according to the present invention for use in a method for treatment of disrupted osteoclast function, of bone resorption disorder and of cancers of hematopoietic origin. Preferably, present invention provides the pharmaceutical composition comprising idelalisib for use in a method of chronic lymphocytic leukemia (CLL) or follicular lymphoma (FL).

### Examples

### Comparative example 1

| Component | Amount per FDF(mg) | Portion |
|---|---|---|
| amorphous idelalisib | 150 | 75% |
| low-substituted hydroxypropyl cellulose | 10 | 5% |
| microcrystalline cellulose | 20 | 10% |
| croscarmellose | 6 | 3% |
| sodium starch glycolate | 12 | 6% |
| magnesium stearate | 2 | 1% |
| Total | 200 | / |

FDF comprising amorphous idelalisib and pharmaceutical excipients that are used in the reference product on the market was prepared by direct compression. Amorphous idelalisib was mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 37% of the total amount in the first 10 minutes in 0.1 M HCl and 99% of the total amount in 60 minutes in a buffer with pH of about 6.4.

### Comparative example 2

| Component | Amount per FDF(mg) | Portion |
|---|---|---|
| crystalline idelalisib | 150 | 75% |
| low-substituted hydroxypropyl cellulose | 10 | 5% |
| microcrystalline cellulose | 20 | 10% |
| croscarmellose | 6 | 3% |
| sodium starch glycolate | 12 | 6% |
| magnesium stearate | 2 | 1 % |
| Total | 200 | / |

FDF comprising crystalline idelalisib and pharmaceutical excipients that are used in the reference product on the market was prepared by direct compression. The amount of excipients and manufacturing procedure were comparable to the reference example 2. Crystalline idelalisib was mixed with low-substituted hydroxypropyl cellulose, microcrystalline cellulose, croscarmellose, sodium starch glycolate and magnesium stearate. Final mixture was compressed into tablets by a tablet press to obtain FDF.

Crystalline idelalisib was confirmed by X-ray measurements of the FDF.

Idelalisib release from the FDF was 100% of the total amount in the first 10 minutes in 0.1 M HCl and 41 % of the total amount in 60 minutes in a buffer with pH of about 6.4.

### Example 1

| Component | Amount per FDF(mg) | Portion |
|---|---|---|
| amorphous idelalisib | 150 | 75% |
| hydroxypropyl cellulose | 20 | 10% |
| low-substituted hydroxypropyl cellulose | 10 | 5% |
| microcrystalline cellulose | 5 | 2,5% |
| croscarmellose | 5 | 2,5% |
| sodium starch glycolate | 8 | 4% |
| magnesium stearate | 2 | 1% |
| Total | 200 | / |

FDF comprising amorphous idelalisib, hydroxypropyl cellulose and pharmaceutical excipients that are used in the reference product on the market was prepared by direct compression. Amorphous idelalisib was mixed with hydroxypropyl cellulose, low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 75 % of the total amount in the first 10 minutes in 0.1 M HCl and 72% of the total amount in 60 minutes in a buffer with pH of about 6.4.

### Example 2

| Component | Amount per FDF(mg) | Portion |
|---|---|---|
| amorphous idelalisib | 150 | 75% |
| phenylalanin | 20 | 10% |
| low-substituted hydroxypropyl cellulose | 10 | 5% |
| microcrystalline cellulose | 5 | 2,5% |
| croscarmellose | 5 | 2,5% |
| sodium starch glycolate | 8 | 4% |
| magnesium stearate | 2 | 1% |
| Total | 200 | / |

FDF comprising amorphous idelalisib, phenylalanin and pharmaceutical excipients that are used in the reference product on the market was prepared by direct compression. Amorphous idelalisib was mixed with phenylalanin, low-substituted hydroxypropyl cellulose, microcrystalline cellulose, croscarmellose, sodium starch glycolate and magnesium stearate. Final mixture was compressed tablets by a tablet press to obtain FDF.

Amorphous idelalisib was confirmed by X-ray measurements of the FDF.

Idelalisib release from the FDF was 102 % of the total amount in the first 10 minutes in 0.1 M HCl and 66% of the total amount in 60 minutes in a buffer with pH of about 6.4.

### Example 3

| Component | Amount per FDF(mg) | Portion |
|---|---|---|
| amorphous idelalisib | 150 | 75% |
| hydroxypropyl cellulose | 10 | 5% |
| low-substituted hydroxypropyl cellulose | 5 | 2,5% |
| microcrystalline cellulose | 5 | 2,5% |
| croscarmellose | 10 | 5% |
| sodium starch glycolate | 18 | 9% |
| magnesium stearate | 2 | 1% |
| Total | 200 | / |

FDF comprising amorphous idelalisib and hydroxypropyl cellulose was prepared by granulating amorphous idelalisib with hydroxypropyl cellulose dissolved in water, following the direct compression with pharmaceutical excipients that are used in the reference product on the market. Hydroxypropyl cellulose was dissolved in water and amorphous idelalisib was granulated with the resulting solution. The resulting wet granulate was dried and mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 97 % of the total amount in the first 10 minutes in 0.1 M HCl and 86% of the total amount in 60 minutes in a buffer with pH of about 6.4.

### Example 4

| Component | Amount per FDF(mg) | Portion |
|---|---|---|
| amorphous idelalisib | 150 | 75% |
| Hydroxypropyl methyl cellulose | 10 | 5% |
| low-substituted hydroxypropyl cellulose | 5 | 2,5% |
| microcrystalline cellulose | 7 | 3,5% |
| croscarmellose | 9 | 4,5% |
| sodium starch glycolate | 17 | 8,5% |
| magnesium stearate | 2 | 1% |
| Total | 200 | / |

FDF comprising amorphous idelalisib and hydroxypropyl methyl cellulose was prepared by granulating amorphous idelalisib with hydroxypropyl methyl cellulose dissolved in water, following the direct compression with pharmaceutical excipients that are used in the reference product on the market. Hydroxypropyl methyl cellulose was dissolved in water and amorphous idelalisib was granulated with the resulting solution. The resulting wet granulate was dried and mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 96 % of the total amount in the first 10 minutes in 0.1 M HCl and 63 % of the total amount in 60 minutes in a buffer with pH of about 6.4.

### Comparative Example 3

| Component | Amount per FDF(mg) | Portion |
|---|---|---|
| amorphous idelalisib | 150 | 75% |
| polyvinylpyrrolidone | 10 | 5% |
| low-substituted hydroxypropyl cellulose | 5 | 2,5% |
| microcrystalline cellulose | 7 | 3,5% |
| croscarmellose | 9 | 4,5% |
| sodium starch glycolate | 17 | 8,5% |
| magnesium stearate | 2 | 1% |
| Total | 200 | / |

FDF comprising amorphous idelalisib and polyvinylpyrrolidone was prepared by granulating amorphous idelalisib with polyvinylpyrrolidone dissolved in water, following the direct compression with pharmaceutical excipients that are used in the reference product on the market. Polyvinylpyrrolidone was dissolved in water and amorphous idelalisib was granulated with the resulting solution. The resulting wet granulate was dried and mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 38 % of the total amount in the first 10 minutes in 0.1 M HCl and 82 % of the total amount in 60 minutes in a buffer with pH of about 6.4.

### Example 5

| Component | Amount per FDF(mg) | Portion |
|---|---|---|
| amorphous idelalisib | 150 | 75% |
| hydroxypropyl cellulose | 20 | 10% |
| low-substituted hydroxypropyl cellulose | 10 | 5% |
| microcrystalline cellulose | 5 | 2,5% |
| croscarmellose | 5 | 2,5% |
| sodium starch glycolate | 8 | 4% |
| magnesium stearate | 2 | 1% |
| Total | 200 | / |

FDF comprising amorphous idelalisib and hydroxypropyl cellulose was prepared by grinding amorphous idelalisib with hydroxypropyl cellulose with pestle and mortar to form a homogenous mixture, following the direct compression with pharmaceutical excipients that are used in the reference product on the market. Hydroxypropyl cellulose and amorphous idelalisib were grinded with pestle and mortar to form a homogenous mixture. The resulting homogenous mixture was mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 97 % of the total amount in the first 10 minutes in 0.1 M HCl and 93% of the total amount in 60 minutes in a buffer with pH of about 6.4.

### Example 6

| Component | Amount per FDF(mg) | Portion |
|---|---|---|
| amorphous idelalisib | 150 | 75% |
| mannitol | 20 | 10% |
| low-substituted hydroxypropyl cellulose | 10 | 5% |
| microcrystalline cellulose | 5 | 2,5% |
| croscarmellose | 5 | 2,5% |
| sodium starch glycolate | 8 | 4% |
| magnesium stearate | 2 | 1% |
| Total | 200 | / |

FDF comprising amorphous idelalisib and mannitol was prepared by grinding amorphous idelalisib with mannitol with pestle and mortar to form a homogenous mixture, following the direct compression with pharmaceutical excipients that are used in the reference product on the market. Mannitol and amorphous idelalisib were grinded with pestle and mortar to form a homogenous mixture. The resulting homogenous mixture was mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 88 % of the total amount in the first 10 minutes in 0.1 M HCl and 88 % of the total amount in 60 minutes in a buffer with pH of about 6.4.

### Comparative Example 4

| Component | Amount per FDF(mg) | Portion |
|---|---|---|
| amorphous idelalisib | 150 | 75% |
| sodium lauryl sulfate | 20 | 10% |
| low-substituted hydroxypropyl cellulose | 10 | 5% |
| microcrystalline cellulose | 5 | 2,5% |
| croscarmellose | 5 | 2,5% |
| sodium starch glycolate | 8 | 4% |
| magnesium stearate | 2 | 1% |
| Total | 200 | / |

FDF comprising amorphous idelalisib and sodium lauryl sulfate was prepared by grinding amorphous idelalisib with sodium lauryl sulfate with pestle and mortar to form a homogenous mixture, following the direct compression with pharmaceutical excipients that are used in the reference product on the market. Sodium lauryl sulfate and amorphous idelalisib were grinded with pestle and mortar to form a homogenous mixture. The resulting homogenous mixture was mixed with low-substituted hydroxypropyl cellulose, microcrystalline cellulose, croscarmellose, sodium starch glycolate and magnesium stearate. Final mixture was compressed into tablets by a tablet press to obtain FDF.

Amorphous idelalisib was confirmed by X-ray measurements of the FDF.

Idelalisib release from the FDF was 10 % of the total amount in the first 10 minutes in 0.1 M HCl and 26 % of the total amount in 60 minutes in a buffer with pH of about 6.4.

### Comparative Example 5

Comparative Example 4 was repeated while replacing sodium lauryl sulfate by another surfactant, a poloxamer (poloxamer type 188).

Idelalisib release from the FDF was 29 % of the total amount in the first 10 minutes in 0.1 M HCl and 11 % of the total amount in 60 minutes in a buffer with pH of about 6.4.

### Release from FDF tests:

A measure of release of the idelalisib from the FDF was performed in controlled laboratory environment by using standard paddle apparatus (Apparatus 2) with agitation of 50 RPM. FDF according to the examples were subjected to acidic media, 0.1 M HCl that simulated gastric condition and to media with neutral pH, buffer with pH of about 6.4 that simulated post-gastric conditions. For gastric conditions testing time point of 10 minutes was chosen due to quick removal of pharmaceutical composition from the stomach. For the post-gastric conditions, testing time point of 60 minutes was chosen due to relatively long residence of pharmaceutical composition in the small intestine. Release of idelalisib from FDF was expressed in the percentage of a total amount of idelalisib release within the selected time points. More specifically the release of idelalisib from FDF in acidic media was expressed in percent of total amount of idelalisib released within 10 min in 900ml 0.1 M HCl and the release of idelalisib from FDF in media with neutral pH was expressed in percent of total amount of idelalisib released within 60 min in 900ml FaSSIF buffer with pH of about 6.4.

### Confirming amorphous idelalisib

The amorphous idelalisib was characterized by an X-ray powder diffraction pattern, showing a plain halo with no well-defined peaks as shown in Fig.1 of WO 2015/092810.

## Claims

1. Pharmaceutical composition of idelalisib in the form of an FDF comprising idelalisib, or a salt or a prodrug or a solvate thereof, and one or more pharmaceutically acceptable excipient, wherein said idelalisib, or a salt or a prodrug or a solvate thereof, is in amorphous form, and wherein the pharmaceutical composition provides for an idelalisib release property defined by at least 60% of the total amount of idelalisib release from FDF when measured in each of acidic media and media with neutral pH.

2. The pharmaceutical composition according to claim 1, wherein said idelalisib release property defined by at least 60% of the total amount of idelalisib release from FDF in each of acidic media and media with neutral pH is respectively met after 10 minutes in 0.1 HCl and after 60 minutes at pH of about 6.4.

3. The pharmaceutical composition according to claim 1 or 2, wherein said idelalisib release property to be met in each of said acidic media and neutral media is defined by at least 70%, preferably at least 80% of the total amount of idelalisib release from FDF.

4. The pharmaceutical composition according to anyone of the preceding claims, wherein said idelalisib release property of the pharmaceutical composition is provided for by selecting the pharmaceutically acceptable excipients from the groups consisting of cellulose derivatives, aromatic amino acids, and polyols.

5. The pharmaceutical composition according to claim 4, wherein the cellulose derivative excipient is selected from hydroxypropylcellulose, hydroxypropylmethylcellulose, methylcellulose, ethylcellulose, hydroxyethylcellulose, carboxymethylcellulose, preferably hydroxypropyl cellulose and hydroxypropylmethyl cellulose; the aromatic amino acid excipient is phenylalanine, and the polyol excipient is mannitol.

6. The pharmaceutical composition according to anyone of the preceding claims, wherein said idelalisib release property of the pharmaceutical composition is provided for by containing a mechanically intimately mixed association of amorphous idelalisib, or a salt or a prodrug or a solvate thereof, with the one or more pharmaceutically acceptable excipient.

7. The pharmaceutical composition according to claim 6, wherein the mechanically intimately mixed association is formed by high-shear mixing, preferably by co-milling of amorphous idelalisib, or a salt or a prodrug or a solvate thereof, with the one or more pharmaceutically acceptable excipient.

8. The pharmaceutical composition according to any of the proceeding claims, comprising further pharmaceutically acceptable excipients selected from disintegrants, binders, fillers, and lubricants.

9. The pharmaceutical composition according to any of preceding claims, comprising relative to the total weight amount of the formulation:
(i) about 10 - 85% amorphous idelalisib or a salt or a prodrug or a solvate thereof,
(ii) about 1 - 30% excipients selected from cellulose derivatives, aromatic amino acids and polyols, wherein preferably the cellulose derivative is selected from hydroxypropyl cellulose and hydrohypropylmethyl cellulose; the aromatic amino acid preferably is phenylalanine; and polyol preferably is mannitol.
(iii) about 0 - 30% disintegrant, preferably croscarmellose
(iv) about 0 - 30% binder, preferably low-substituted hydroxypropyl cellulose and sodium starch glycolate,
(v) about 0 - 30% filler, preferably microcrystalline cellulose,
(vi) about 0.2 - 3% lubricant, preferably magnesium stearate.

10. A pharmaceutical composition according to any one of the preceding claims, obtained by direct compression.

11. A granulate obtained by wet granulating of amorphous idelalisib, or a salt or a prodrug or a solvate thereof, or a mixture of amorphous idelalisib, or a salt or a prodrug or a solvate thereof, and at least one pharmaceutically acceptable excipient, with a liquid or a solution of pharmaceutically acceptable excipient(s).

12. A mixture of amorphous idelalisib, or a salt or a prodrug or a solvate thereof, co-milled with one or more pharmaceutically acceptable excipient selected from the groups consisting of cellulose derivatives and polyols.

13. A process for preparing a pharmaceutical composition according to anyone of claims 1 to 10 comprising
(i) mixing the amorphous idelalisib, or a salt or a prodrug or a solvate thereof, with said pharmaceutically acceptable excipients under a condition of absence of any liquid;
(ii) compressing the mixture; or
(i') mechanically intimately mixing, preferably grinding, amorphous idelalisib, or a salt or a prodrug or a solvate thereof, with at least one pharmaceutically acceptable excipient to form a homogenous mixture, wherein the at least one pharmaceutically acceptable excipient is preferably selected from the groups consisting of cellulose derivatives, aromatic amino acids, and polyols, more preferably the cellulose derivative is selected from hydroxypropylcellulose, hydroxypropylmethylcellulose, methylcellulose, ethylcellulose, hydroxyethylcellulose, carboxymethylcellulose, in particular is selected from hydroxypropyl cellulose and hydroxypropylmethyl cellulose, the aromatic amino acid excipient preferably is phenylalanine; and the polyol excipient preferably is mannitol;
(ii') mixing the thus obtained mixture with pharmaceutically acceptable excipient selected from disintegrants, binders, fillers, and lubricants under a condition of absence of any liquid;
(iii') compressing the mixture; or
(i") granulating amorphous idelalisib, or a salt or a prodrug or a solvate thereof, or a mixture of amorphous idelalisib, or a salt or a prodrug or a solvate thereof, and at least one pharmaceutically acceptable excipient with a liquid or a solution of pharmaceutically acceptable excipient(s) to form wet granulate, wherein the at least one pharmaceutically acceptable excipient is selected from the groups consisting of cellulose derivatives, more preferably the cellulose derivative is selected from hydroxypropylcellulose, hydroxypropylmethylcellulose, methylcellulose, ethylcellulose, hydroxyethylcellulose and carboxymethylcellulose, in particular is selected from hydroxypropylcelulose and hydrohypropylmethylcelulose;
(ii") mixing the mixture obtained in (i") with at least one further pharmaceutically acceptable excipient selected from disintegrants, binders, fillers, and lubricants under a condition of absence of any liquid;
(iii") compressing the mixture.

14. The process according to claim 13, wherein the mixture is compressed and tableted into tablet cores, optionally wherein the tablet cores are further coated with coating containing polymer selected from the group consisting of polyvinyl alcohol, hydroxypropyl methylcellulose, hydroxypropyl cellulose.

15. The pharmaceutical composition according to anyone of claims 1 to 10 for use in the method of treatment of disrupted osteoclast function, bone resorption disorder and of cancers of hematopoietic origin.
